# DEMANDE DE BREVET EUROPEEN

(11) **EP 3 498 371 A1**
(43) Date de publication de la demande: **19.06.2019**
(21) Numéro de dépôt: 18207040.9
(22) Date de dépôt: 19.11.2018
(51) Int. Cl.: B01J 29/74, B01J 29/76, B01J 29/78, C07C 5/27, B01J 37/00

(54) **PROCEDE DE PREPARATION D'UN CATALYSEUR A BASE D'IZM-2 A PARTIR D'UNE SOLUTION COMPRENANT DES PRECURSEURS SPECIFIQUES ET UTILISATION POUR L'ISOMERISATION DE CHARGES PARAFFINIQUES**

(30) Priorité: 06.12.2017 FR 1761691
(71) Demandeur: IFP Energies nouvelles, 92852 Rueil-Malmaison Cedex (FR)
(72) Inventeur: BOUCHY, Christophe, 69007 LYON (FR)
(74) Mandataire: IFP Energies nouvelles

(57) **Abrégé**

La présente invention concerne un procédé de préparation d'un catalyseur bifonctionnel mettant en oeuvre une zéolithe IZM-2, une fonction hydrogénante et une matrice. Le procédé de préparation selon l'invention permet à la fois une localisation préférentielle de ladite fonction hydrogénante sur la surface et/ou dans la microporosité de la zéolithe IZM-2 et une répartition homogène de la fonction hydrogénante dans le catalyseur et de préférence sur la zéolithe IZM-2 grâce à la mise en oeuvre d'une solution d'imprégnation comprenant des précurseurs de métaux nobles spécifiques combinés avec la présence de sels d'ammonium, avec un rapport sel d'ammonium sur métal noble bien précis.

## Description

Afin de répondre à la demande en bases distillats moyens, c'est-à-dire en coupe incorporable au pool kérosène et/ou gazole, diverses méthodes de production de distillats moyens basées sur l'utilisation du pétrole, de gaz naturel ou encore de ressources renouvelables peuvent être mises en oeuvre.

Les bases distillats moyens peuvent ainsi être produites à partir d'une charge paraffinique obtenue à partir d'une charge issue de sources renouvelables, et en particulier d'huiles végétales ou des graisses animales, brutes ou ayant subi un traitement préalable, ainsi que les mélanges de telles charges. En effet, lesdites charges issues de sources renouvelables contiennent des structures chimiques de type triglycérides ou esters ou acides gras libres, la structure et la longueur de chaîne hydrocarbonée de ces derniers étant compatibles avec les hydrocarbures présents dans les distillats moyens. Lesdites charges issues de sources renouvelables produisent, après hydrotraitement, des charges paraffiniques, exemptes de composés soufrés et de composés aromatiques. Ces charges paraffiniques sont typiquement composées de paraffines linéaires ayant un nombre d'atomes de carbone compris entre 9 et 25.

Les bases distillats moyens peuvent aussi être produites à partir de gaz naturel, charbon, ou sources renouvelables par l'intermédiaire du procédé de synthèse de Fischer-Tropsch. En particulier la synthèse de Fischer-Tropsch dite basse température utilisant des catalyseurs au cobalt permet de produire des composés essentiellement paraffiniques linéaires ayant un nombre d'atomes de carbone très variable, typiquement de 1 à 100 atomes de carbone voire plus. Des étapes de séparation peuvent permettre de récupérer des charges paraffiniques ayant un nombre d'atomes de carbone compris entre 9 et 25.

Toutefois, ces bases distillats moyens obtenues après hydrotraitement des huiles végétales ou après le procédé de synthèse de Fischer-Tropsch basse température ne peuvent généralement pas être incorporées telles quelles au pool kérosène ou gazole notamment en raison de propriétés à froid insuffisantes. En effet, les paraffines de haut poids moléculaire qui sont linéaires ou très faiblement branchées et qui sont présentes dans ces bases distillats moyens conduisent à des points d'écoulement hauts et donc à des phénomènes de figeage pour des utilisations à basse température. Par exemple, le point d'écoulement d'un hydrocarbure linéaire contenant 20 atomes de carbone par molécule et dont le température d'ébullition égale à 340°C environ c'est à dire typiquement comprise dans la coupe distillats moyens, est de +37°C environ ce qui rend son utilisation impossible, la spécification étant de -15°C pour le gazole. Afin de diminuer les valeurs des points d'écoulement, ces paraffines linéaires ou très peu branchées doivent être entièrement ou partiellement éliminées.

Cette opération peut s'effectuer par extraction par des solvants tels que le propane ou la méthyl-éthyl cétone, on parle alors de déparaffinage au propane ou à la méthyl éthyl-cétone (MEK). Cependant, ces techniques sont coûteuses, longues et pas toujours aisées à mettre en oeuvre.

Le craquage sélectif des chaînes paraffiniques linéaires les plus longues qui conduit à la formation de composés de poids moléculaire plus faible dont une partie peut être éliminée par distillation constitue une solution pour diminuer les valeurs des points d'écoulement. Compte tenu de leur sélectivité de forme les zéolithes sont parmi les catalyseurs les plus utilisés pour ce type de procédé. Le catalyseur le plus utilisé dans la catégorie déparaffinage par craquage sélectif est la zéolithe ZSM-5, de type structural MFI, qui présente une porosité tridimensionnelle, avec des pores moyens (ouverture à 10 atomes d'oxygènes 10MR). Toutefois, le craquage occasionné dans de tels procédés conduit à la formation de quantités importantes de produits de poids moléculaires plus faibles, tels que du butane, propane, éthane et méthane, ce qui réduit considérablement le rendement en produits recherchés.

Une autre solution pour améliorer la tenue à froid consiste à isomériser les paraffines linéaires longues en minimisant au maximum le craquage. Ceci peut être réalisé par la mise en oeuvre de procédé d'hydroisomérisation employant des catalyseurs bifonctionnels. Les catalyseurs bifonctionnels mettent en jeu une phase acide de Bronsted (par exemple une zéolithe) et une phase hydro/déshydrogénante (par exemple du platine) et généralement une matrice (par exemple de l'alumine). Le choix approprié de la phase acide permet de favoriser l'isomérisation des paraffines linéaires longues et de minimiser le craquage. Ainsi la sélectivité de forme des zéolithes monodimensionnelles à pores moyens (10MR) comme les zéolithes ZSM-22, ZSM-23, NU-10, ZSM-48, ZBM-30 rend leur utilisation particulièrement adaptée pour obtenir des catalyseurs sélectifs envers l'isomérisation.

Récemment la demanderesse a également découvert que l'utilisation de la zéolithe IZM-2 est également appropriée pour obtenir des catalyseurs sélectifs envers l'isomérisation des paraffines longues.

Toutefois il est bien connu que des facteurs autres que la phase acide ont un impact sur l'activité et la sélectivité d'un catalyseur bifonctionnel. L'hydroisomérisation et l'hydrocraquage des normales paraffines ont ainsi fait l'objet de nombreuses études académiques depuis les travaux originels des années soixante de Weisz ou Coonradt et Garwood. Le mécanisme le plus communément admis implique tout d'abord que la n-paraffine soit déshydrogénée en n-oléfine sur la phase hydro-déshydrogénante puis, après diffusion vers la phase acide, qu'elle soit protonée en ion carbénium. Après réarrangement structural et/ou β-scission, les ions carbéniums désorbent de la phase acide sous forme d'oléfines après déprotonation. Puis, après diffusion vers la phase hydro-déshydrogénante, les oléfines sont hydrogénées pour former les produits de réaction finaux. Lorsqu'une sélectivité maximale en isomérisation est recherchée, il convient de limiter les réactions de craquage sur la phase acide. Il convient alors d'avoir une fonction hydro/déshydrogénante suffisamment active vis-à-vis de la fonction acide pour hydrogéner rapidement les intermédiaires oléfiniques. Lorsque la vitesse globale de réaction est uniquement contrôlée par les étapes catalysées par la fonction acide, le catalyseur bifonctionnel est dit "idéal".

La proximité entre les deux fonctions du catalyseur peut également avoir un impact sur les performances du catalyseur bifonctionnel. Ainsi, Zecevic et coll. (Nature, 2015, 528, 245-254) ont récemment étudié l'impact de la localisation du platine sur les performances en hydroisomérisation de paraffines longues (n-décane, n-nonadécane, pristane) d'un catalyseur bifonctionnel utilisant la zéolithe USY comme phase acide et une matrice alumine. Il est observé que le catalyseur bifonctionnel pour lequel le platine est déposé sur l'alumine est systématiquement plus sélectif en isomérisation que le catalyseur pour lequel le platine est déposé dans la zéolithe. Au vu de ces résultats l'homme de l'art est donc enclin à privilégier une localisation de la fonction hydrogénante sur la matrice alumine plutôt que sur la phase acide pour améliorer la sélectivité en isomérisation. D'un point de vue de l'activité, la localisation du platine sur la matrice alumine a un impact variable selon la paraffine longue considérée : impact positif en ce qui concerne le n-décane, impact marginal en ce qui concerne le n-nonadécane et enfin impact négatif en ce qui concerne le pristane.

Lors de ses travaux effectués pour améliorer la sélectivité en isomérisation de paraffines longues et l'activité de catalyseurs bifonctionnels utilisant de la zéolithe IZM-2 comme fonction acide, la demanderesse a découvert un impact surprenant de la localisation de la fonction hydro/déshydrogénante combinée à la répartition de ladite fonction sur les performances du catalyseur bifonctionnel.

Ainsi, la présente invention concerne un procédé de préparation d'un catalyseur bifonctionnel mettant en oeuvre une zéolithe IZM-2, une fonction hydrogénante et une matrice. Le procédé de préparation selon l'invention permet à la fois une localisation préférentielle de ladite fonction hydrogénante sur la surface et/ou dans la microporosité de la zéolithe IZM-2 et une répartition homogène de la fonction hydrogénante dans le catalyseur et de préférence sur la zéolithe IZM-2 grâce à la mise en oeuvre d'une solution d'imprégnation comprenant des précurseurs de métaux nobles spécifiques combinés avec la présence de sels d'ammonium, avec un rapport sel d'ammonium sur métal noble bien précis.

Une autre objet de la présente invention concerne le catalyseur obtenu par ledit procédé.

Une autre objet de la présente invention concerne un procédé d'isomérisation de charges paraffiniques issues des huiles végétales et/ou animales hydrotraitées ou de la synthèse Fischer-Trospch basse température, ledit procédé mettant en oeuvre ledit catalyseur bifonctionnel.

De manière surprenante, la localisation préférentielle de la fonction hydrogénante comprenant au moins un métal noble du groupe VIII et de préférence du platine sur la surface et/ou dans la microporosité de la zéolithe IZM-2 permet d'obtenir un catalyseur plus sélectif envers la réaction d'isomérisation que lorsque ladite fonction est déposée préférentiellement sur la matrice alumine. Par ailleurs lorsque ladite fonction hydrogénante est déposée préférentiellement sur et/ou dans la microporosité de la zéolithe IZM-2 et de plus répartie de manière homogène au sein du catalyseur l'activité du catalyseur s'en trouve améliorée.

### Résumé de l'invention

La présente invention porte sur un procédé de préparation d'un catalyseur bifonctionnel comprenant une fonction acide constituée par de la zéolithe IZM-2, une fonction hydrogénante comprenant au moins un métal noble du groupe VIII de la classification périodique, choisi parmi le platine et le palladium et une matrice, ledit procédé comprenant au moins les étapes suivantes :
i) une étape de préparation du support du catalyseur par mise en forme de la zéolithe IZM-2 avec une matrice de sorte que le pourcentage poids de la zéolithe soit avantageusement compris entre 1 et 50% par rapport au poids du support,
ii) une étape de dépôt d'au moins un métal noble du groupe VIII de la classification périodique par imprégnation du support préparé à l'étape i) par une solution aqueuse comprenant au moins les composés suivants :

- au moins un composé ammoniaqué choisi parmi les sels de platine (II) tétramines de formule Pt(NH₃)₄(OH)₂, Pt(NH₃)₄(NO₃)₂ ou Pt(NH₃)₄X₂, les sels de platine (IV) hexamines de formule Pt(NH₃)₆X₄; les sels de platine (IV) halogénopentamines de formule (PtX(NH₃)₅)X₃; les sels de platine N-tétrahalogénodiamines de formule PtX₄(NH₃)₂; et les composés halogénés de formule H(Pt(acac)₂X); les sels de palladium (II) Pd(NH₃)₄SO₄ ou Pd(NH₃)₄X₂, dans lesquels X est un halogène choisi parmi le chlore, le fluor, le brome et l'iode, X étant de préférence le chlore, et "acac" représente le groupe acétylacétonate (de formule brute C₅H₇O₂), composé dérivé de l'acétylacétone,
- et au moins un sel d'ammonium choisi parmi le nitrate d'ammonium NH₄NO₃, le chlorure d'ammonium NH₄Cl, l'hydroxyde d'ammonium NH₄OH, le bicarbonate d'ammonium NH₄HCO₃, l'acétate d'ammonium NH₄H₃C₂O₂ seul ou en mélange,
- le rapport molaire entre le sel d'ammonium et le métal noble étant compris entre 0,1 et 400.

Un avantage de la présente invention est de fournir un procédé de préparation d'un catalyseur bifonctionnel comprenant une phase acide à base de zéolithe IZM-2 et une fonction hydrogénante à base de métaux nobles du groupe VIII, qui grâce à l'utilisation d'une solution d'imprégnation comprenant des précurseurs de métaux nobles spécifiques combinés avec la présence de sels d'ammonium, avec un rapport sel d'ammonium sur métal noble bien précis, permet à la fois l'obtention d'une localisation préférentielle de ladite fonction hydrogénante sur la surface et/ou dans la microporosité de la zéolithe IZM-2 et une répartition homogène de la fonction hydrogénante dans le catalyseur.

Un avantage de la présente invention est de fournir un catalyseur obtenu par ledit procédé, ledit catalyseur étant caractérisé par une localisation préférentielle de ladite fonction hydrogénante sur la surface et/ou dans la microporosité de la zéolithe IZM-2 et une répartition homogène de la fonction hydrogénante dans le catalyseur.

Un autre avantage de la présente invention est de fournir un procédé d'isomérisation de charges paraffiniques issues des huiles végétales et/ou animales hydrotraitées ou de la synthèse Fischer-Trospch basse température mettant en oeuvre ledit catalyseur bifonctionnel ainsi obtenu permettant d'une part l'obtention d'une meilleure sélectivité en distillats moyens grâce à la localisation de la fonction hydrogénante sur la surface et/ou dans la microporosité de la zéolithe IZM-2, et également une meilleure activité, dans le cas ou en plus d'être localisé préférentiellement sur la zéolithe IZM-2, la fonction hydrogénante est également répartie de manière homogène sur ledit catalyseur.

### Description détaillée de l'invention

Conformément à l'invention, la présente invention concerne un procédé de préparation d'un catalyseur bifonctionnel comprenant une fonction acide constituée par de la zéolithe IZM-2, une fonction hydrogénante comprenant au moins un métal noble du groupe VIII de la classification périodique choisi parmi le platine et le palladium seul ou en mélange et une matrice.

Le catalyseur préparé selon l'invention comprend la zéolithe IZM-2 qui constitue la fonction acide dudit catalyseur. La zéolithe IZM-2 présente une structure cristalline.

La zéolithe IZM-2 est un solide microporeux cristallisé présentant une structure cristalline décrite dans la demande de brevet FR 2 918 050. Le procédé de préparation de la zéolithe IZM-2 est également décrit dans ladite demande.

Ledit solide IZM-2 présente une composition chimique exprimée sur une base anhydre, en termes de moles d'oxydes, définie par la formule générale suivante : XO₂ : aY₂O₃ : bM₂/nO, dans laquelle X représente au moins un élément tétravalent, Y représente au moins un élément trivalent et M est au moins un métal alcalin et/ou un métal alcalino-terreux de valence n.

X est préférentiellement choisi parmi le silicium, le germanium, le titane et le mélange d'au moins deux de ces éléments tétravalents, très préférentiellement X est le silicium et Y est préférentiellement choisi parmi l'aluminium, le bore, le fer, l'indium et le gallium, très préférentiellement Y est l'aluminium. M est préférentiellement choisi parmi le lithium, le sodium, le potassium, le calcium, le magnésium et le mélange d'au moins deux de ces métaux et très préférentiellement M est le sodium. De manière préférée, X représente le silicium, le solide cristallisé IZM-2 selon l'invention est alors un solide entièrement silicique lorsque l'élément Y est absent de la composition dudit solide IZM-2. Il est également avantageux d'employer comme élément X un mélange de plusieurs éléments X, en particulier un mélange de silicium avec un autre élément X choisi parmi le germanium et le titane, de préférence le germanium. Ainsi, lorsque le silicium est présent en mélange avec un autre élément X, le solide cristallisé IZM-2 selon l'invention est alors un métallosilicate cristallisé présentant un diagramme de diffraction des rayons X identique à celui décrit dans le tableau 1 lorsqu'il se trouve sous sa forme calcinée. De manière encore plus préférée et en présence d'un élément Y, X étant le silicium et Y étant l'aluminium : le solide cristallisé IZM-2 selon l'invention est alors un aluminosilicate.

De préférence, la zéolithe IZM-2 est sous forme aluminosilicate.

De préférence, le rapport molaire du nombre d'atomes de silicium sur le nombre d'atomes d'aluminium Si/AI est inférieur à 200, de préférence inférieure à 150, de manière très préférée inférieure à 120.

La zéolithe IZM-2 entrant dans la composition du support du catalyseur préparé selon l'invention est avantageusement échangée par au moins un traitement par une solution d'au moins un sel d'ammonium de manière à obtenir la forme ammonium de la zéolithe IZM-2 qui une fois calcinée conduit à la forme acide (H+) de ladite zéolithe IZM-2. Cette étape d'échange peut être effectuée à toute étape de la préparation du catalyseur, c'est-à-dire après l'étape de préparation de la zéolithe IZM-2, après l'étape de mise en forme de la zéolithe IZM-2 avec une matrice, ou encore après l'étape d'introduction du métal hydro-déshydrogénant.

Ladite zéolithe IZM-2 entrant dans la composition du support du catalyseur utilisé dans le procédé selon l'invention est avantageusement au moins en partie, de préférence pratiquement totalement, sous forme acide, c'est-à-dire sous forme acide (H+).

Selon l'invention, le catalyseur préparé comprend au moins une matrice. Ladite matrice peut avantageusement être amorphe ou cristallisée.

De préférence, ladite matrice est avantageusement choisie dans le groupe formé par l'alumine, la silice, la silice-alumine, les argiles, l'oxyde de titane, l'oxyde de bore et la zircone, pris seuls ou en mélange ou bien on peut choisir également les aluminates. De préférence, l'alumine est utilisée comme matrice. De manière préférée, ladite matrice contient de l'alumine sous toutes ses formes connues de l'homme du métier, telles que par exemple les alumines de type alpha, gamma, êta, delta. Lesdites alumines diffèrent par leur surface spécifique et leur volume poreux.

Le mélange de la matrice et de la zéolithe IZM-2 mis en forme constitue le support du catalyseur.

### Etape i)

Conformément à l'invention, le procédé comprend une étape i) de préparation du support du catalyseur par mise en forme de la zéolithe IZM-2 avec une matrice de sorte que le pourcentage poids de la zéolithe soit avantageusement compris entre 1 et 50% par rapport au poids du support.

### Mise en forme

Le support du catalyseur utilisé dans le procédé selon l'invention peut avantageusement être mis en forme par toute technique connue de l'homme du métier. La mise en forme peut avantageusement être réalisée par exemple par extrusion, par pastillage, par la méthode de la coagulation en goutte ("oil-drop"), par granulation au plateau tournant ou par toute autre méthode bien connue de l'homme du métier. Les supports ainsi obtenus peuvent se présenter sous différentes formes et dimensions. De préférence, l'étape i) est mise en oeuvre par malaxage - extrusion.

Lors de la mise en forme du support par malaxage puis extrusion, ladite zéolithe IZM-2 peut être introduite au cours de la mise en solution ou en suspension des composés d'alumine ou précurseurs d'alumine tels que la boéhmite par exemple. Ladite zéolithe IZM-2 peut être, sans que cela soit limitatif, par exemple sous forme de poudre, poudre broyée, suspension, suspension ayant subi un traitement de désagglomération. Ainsi, par exemple, ladite zéolithe peut avantageusement être mise en suspension acidulée ou non à une concentration ajustée à la teneur finale en IZM-2 visée dans le catalyseur selon l'invention. Cette suspension appelée couramment une barbotine est alors mélangée avec les composés d'alumine ou précurseurs d'alumine.

Par ailleurs, l'utilisation d'additifs peut avantageusement être mise en oeuvre pour faciliter la mise en forme et/ou améliorer les propriétés mécaniques finales des supports comme cela est bien connu par l'homme du métier. A titre d'exemple d'additifs, on peut citer notamment la cellulose, la carboxyméthyl-cellulose, la carboxy-éthyl-cellulose, du tall-oil (huile de tall), les gommes xanthaniques, des agents tensio-actifs, des agents floculants comme les polyacrylamides, le noir de carbone, les amidons, l'acide stéarique, l'alcool polyacrylique, l'alcool polyvinylique, des biopolymères, le glucose, les polyéthylènes glycols, etc.

On peut avantageusement ajouter ou retirer de l'eau pour ajuster la viscosité de la pâte à extruder. Cette étape peut avantageusement être réalisée à tout stade de l'étape de malaxage.

Pour ajuster la teneur en matière solide de la pâte à extruder afin de la rendre extrudable, on peut également ajouter un composé majoritairement solide et de préférence un oxyde ou un hydrate. On utilise de manière préférée un hydrate et de manière encore plus préférée un hydrate d'aluminium. La perte au feu de cet hydrate est avantageusement supérieure à 15%.

L'extrusion de la pâte issue de l'étape de malaxage peut avantageusement être réalisée par n'importe quel outil conventionnel, disponible commercialement. La pâte issue du malaxage est avantageusement extrudée à travers une filière, par exemple à l'aide d'un piston ou d'une mono-vis ou double vis d'extrusion. L'extrusion peut avantageusement être réalisée par toute méthode connue de l'homme de métier.

Les supports du catalyseur préparés dans l'étape i) selon l'invention sont en général sous la forme d'extrudés cylindriques ou polylobés tels que bilobés, trilobés, polylobés de forme droite ou torsadée, mais peuvent éventuellement être fabriqués et employés sous la forme de poudres concassées, de tablettes, d'anneaux, de billes et/ou de roues. De préférence, les supports du catalyseur selon l'invention ont la forme de sphères ou d'extrudés. Avantageusement le support se présente sous forme d'extrudés d'un diamètre compris entre 0,5 et 5 mm et plus particulièrement entre 0,7 et 2,5 mm. Les formes peuvent être cylindriques (qui peuvent être creuses ou non) et/ou cylindriques torsadés et/ou multilobées (2, 3, 4 ou 5 lobes par exemple) et/ou anneaux. La forme multilobée est avantageusement utilisée de manière préférée.

### Séchage

Le support ainsi obtenu à l'issu de l'étape i) de mise en forme peut ensuite être avantageusement soumis à une étape de séchage. Ladite étape de séchage est avantageusement effectuée par toute technique connue de l'homme du métier.

De préférence, le séchage est effectué sous flux d'air. Ledit séchage peut également être effectué sous flux de tout gaz oxydant, réducteur ou inerte. De préférence, le séchage est avantageusement effectué à une température comprise entre 50 et 180°C, de manière préférée entre 60 et 150°C et de manière très préférée entre 80 et 130°C.

### Calcination

Ledit support, éventuellement séché, subit ensuite de préférence une étape de calcination.

Ladite étape de calcination est avantageusement réalisée en présence d'oxygène moléculaire, par exemple en effectuant un balayage d'air, à une température avantageusement supérieure à 200°C et inférieure ou égale à 1100°C. Ladite étape de calcination peut avantageusement être effectuée en lit traversé, en lit léché ou en atmosphère statique. Par exemple, le four utilisé peut être un four rotatif tournant ou être un four vertical à couches traversées radiales. De préférence, ladite étape de calcination est effectuée entre plus d'une heure à 200°C à moins d'une heure à 1100°C. Pour le support mis en forme et éventuellement séché, la calcination peut avantageusement être opérée en présence de vapeur d'eau et/ou en présence d'une vapeur acide ou basique. Par exemple, la calcination peut être réalisée sous pression partielle d'ammoniaque.

### Traitements post-calcination

Des traitements post-calcination peuvent éventuellement être effectués, de manière à améliorer les propriétés du support calciné, notamment les propriétés texturales.

Ainsi, le support du catalyseur mis en oeuvre dans le procédé selon la présente invention peut être soumis à un traitement hydrothermal en atmosphère confinée. On entend par traitement hydrothermal en atmosphère confinée un traitement par passage à l'autoclave en présence d'eau à une température supérieure à la température ambiante, de préférence supérieure à 25°C, de préférence supérieure à 30°C.

Au cours de ce traitement hydrothermal, on peut avantageusement imprégner le support, préalablement à son passage à l'autoclave (l'autoclavage étant fait soit en phase vapeur, soit en phase liquide, cette phase vapeur ou liquide de l'autoclave pouvant être acide ou non). Cette imprégnation, préalable à l'autoclavage, peut avantageusement être acide ou non. Cette imprégnation, préalable à l'autoclavage peut avantageusement être effectuée à sec ou par immersion du support dans une solution aqueuse acide. Par imprégnation à sec, on entend mise en contact du support avec un volume de solution inférieur ou égal au volume poreux total du support. De préférence, l'imprégnation est réalisée à sec. L'autoclave est de préférence un autoclave à panier rotatif tel que celui défini dans la demande brevet EP 0 387 109 A. La température pendant l'autoclavage peut être comprise entre 100 et 250°C pendant une période de temps comprise entre 30 minutes et 3 heures.

### Etape ii) : dépôt de la fonction hydro-déshydrogénante

Conformément à l'invention, le dépôt de la fonction hydro-déshydrogénante s'effectue après l'étape de mise en forme i).

Conformément à l'invention, le procédé comprend une étape ii) de dépôt d'au moins un métal noble du groupe VIII de la classification périodique par imprégnation du support préparé à l'étape i) et ayant éventuellement subi une étape de séchage et/ou de calcination et/ou de traitement post-calcination, par une solution aqueuse comprenant au moins les composés suivants :
- au moins un composé ammoniaqué choisi parmi les sels de platine (II) tétramines de formule Pt(NH₃)₄(OH)₂, Pt(NH₃)₄(NO₃)₂ ou Pt(NH₃)₄X₂, les sels de platine (IV) hexamines de formule Pt(NH₃)₆X₄; les sels de platine (IV) halogénopentamines de formule (PtX(NH₃)₅)X₃; les sels de platine N-tétrahalogénodiamines de formule PtX₄(NH₃)₂; et les composés halogénés de formule H(Pt(acac)₂X); les sels de palladium (II) Pd(NH₃)₄SO₄ ou Pd(NH₃)₄X₂, dans lesquels X est un halogène choisi parmi le chlore, le fluor, le brome et l'iode, X étant de préférence le chlore, et "acac" représente le groupe acétylacétonate (de formule brute C₅H₇O₂), composé dérivé de l'acétylacétone,
- et au moins un sel d'ammonium choisi parmi le nitrate d'ammonium NH₄NO₃, le chlorure d'ammonium NH₄Cl, l'hydroxyde d'ammonium NH₄OH, le bicarbonate d'ammonium NH₄HCO₃, l'acétate d'ammonium NH₄H₃C₂O₂ seul ou en mélange,
- le rapport molaire entre le sel d'ammonium et le métal noble étant compris entre 0,1 et 400.

La fonction hydro-déshydrogénante peut avantageusement être introduite avant ou après la calcination du support et de préférence après.

Conformément à l'invention, le support est imprégné par une solution aqueuse. L'imprégnation du support est de préférence effectuée par la méthode d'imprégnation dite "à sec" ou en excès d'une solution, bien connues de l'homme du métier. L'imprégnation peut avantageusement être effectuée en une seule étape par une solution contenant l'ensemble des éléments constitutifs du catalyseur final.

De manière préférée, le dépôt par imprégnation en excès est utilisé, dans les conditions suivantes :
- remplissage du volume poreux du support à imprégner par de l'eau distillée et maturation d'au moins trente minutes ;
- mise en contact du support avec la solution d'imprégnation, le rapport du volume de solution sur la masse de support étant compris entre 2 et 150 ml de solution par gramme de support, de manière préférée entre 4 et 100 ml, de manière très préférée entre 5 et 80 ml, pour une durée comprise entre 5 minutes et 48 heures, de manière préférée entre 15 minutes et 36 heures, de manière très préférée entre 30 minutes et 24 heures, à une température comprise entre 10 et 95°C, de préférence entre 15 et 90°C,de manière très préférée entre 20 et 85°C.

La mise en oeuvre de l'étape ii) par imprégnation du support avec une solution aqueuse comprenant les sels ammoniaqués de métaux spécifiques tels que revendiqués permet l'obtention d'un catalyseur bifonctionnel comprenant une phase acide à base de zéolithe IZM-2 et une fonction hydrogénante à base de métaux nobles du groupe VIII dans lequel le métal du groupe VIII est localisé sur la surface externe des cristaux de la zéolithe IZM-2 et/ou dans la microporosité de la zéolithe IZM-2, c'est-à-dire dans les cristaux de la zéolithe IZM-2.

Conformément à l'invention, le catalyseur bifonctionnel préparé selon l'invention comprend au moins un métal noble du groupe VIII choisi parmi le platine et le palladium, seul ou en mélange et de manière très préférée on choisit le platine.

De préférence, l'étape ii) consiste en le dépôt d'au moins un métal noble, de préférence le platine, par imprégnation du support préparé à l'étape i) par une solution aqueuse comprenant des composés ammoniaqués choisis parmi les sels de platine (II) tétramines de formule Pt(NH₃)₄(OH)₂, Pt(NH₃)₄(NO₃), ou Pt(NH₃)₄X₂, les sels de platine (IV) hexamines de formule Pt(NH₃)₆X₄; les sels de platine (IV) halogénopentamines de formule (PtX(NH₃)₅)X₃; les sels de platine N-tétrahalogénodiamines de formule PtX₄(NH₃)₂; et les composés halogénés de formule H(Pt(acac)₂X); X et « acac » ayant la signification précitée, et de préférence parmi les sels de platine (II) tétramines de formule Pt(NH₃)₄(OH)₂, Pt(NH₃)₄(NO₃), ou Pt(NH₃)₄X₂.

De préférence, ladite étape ii) est réalisée de manière à déposer sur ledit support une teneur en métal noble et de préférence en platine comprise entre 0,01 à 4%, et de préférence entre 0,05 à 2% poids par rapport à la masse totale dudit catalyseur.

La localisation préférentielle du métal noble du groupe VIII dans les cristaux et/ou à la surface externe des cristaux de la zéolithe IZM-2 peut être mise en évidence par microsonde de Castaing. Quelques extrudés sont enrobés de résine (Struers, Ballerup) puis polis et métallisés au carbone. On introduit alors l'échantillon dans un appareil JEOL JXA8100 pour analyser en différents points la composition locale en silicium, aluminium, et platine. A partir de la composition locale en aluminium, silicium et connaissant la composition en silicium de la zéolithe, on peut en déduire le rapport massique Alumine/(IZM-2 + alumine) pour chaque point analysé. On peut ainsi tracer l'évolution de la composition locale en platine en fonction du rapport massique local Alumine/(IZM-2 + alumine) et vérifier la localisation préférentielle du platine sur l'alumine ou sur la zéolithe. Lorsque la composition locale en platine augmente avec le rapport massique local Alumine/(IZM-2 + alumine) alors le platine est préférentiellement localisé sur l'alumine. Lorsque la composition locale en platine diminue avec le rapport massique local Alumine/(IZM-2 + alumine) alors le platine est préférentiellement localisé sur la zéolithe.

Conformément à l'invention la solution d'imprégnation contient également au moins un sel d'ammonium ne contenant pas de métaux nobles, choisi parmi le nitrate d'ammonium NH₄NO₃, le chlorure d'ammonium NH₄Cl, l'hydroxyde d'ammonium NH₄OH, le bicarbonate d'ammonium NH₄HCO₃, l'acétate d'ammonium NH₄H₃C₂O₂ seul ou en mélange et de préférence parmi le nitrate d'ammonium NH₄NO₃, le chlorure d'ammonium NH₄Cl et l'acétate d'ammonium NH₄H₃C₂O₂ seul ou en mélange.

Selon l'invention, les concentrations des différentes espèces en solution sont telles que le rapport molaire entre le sel d'ammonium et le métal noble est compris entre 0,1 et 400, de préférence entre 0,2 et 200, de manière très préférée entre 0,3 et 150.

Le rapport molaire entre le sel d'ammonium et le platine est choisi de manière à obtenir une répartition homogène du métal noble et de préférence le platine, dans le catalyseur et de préférence sur la zéolithe IZM-2.

Les concentrations en platine dans la solution d'imprégnation sont ajustées de manière à obtenir la teneur désirée en métal noble dans le catalyseur final.

Ainsi, selon l'invention, la solution d'imprégnation contient à la fois les composés contenant les précurseurs spécifiques de métaux nobles et des sels d'ammonium ne contenant pas de métaux nobles dans les proportions telles que revendiquées. Ceci permet à la fois une localisation préférentielle de ladite fonction hydrogénante sur la surface et/ou dans la microporosité de la zéolithe IZM-2 et une répartition homogène de la fonction hydrogénante dans le catalyseur et de préférence sur la zéolithe.

La microsonde de Castaing permet de vérifier si un élément, en l'occurrence le platine, est réparti de manière homogène au sein du catalyseur, par l'intermédiaire du calcul d'un coefficient de répartition (cf. L. Sorbier, Determining the Distribution of Métal by Electron Probe Micro Analysis, in: H. Toulhoat, P. Raybaud (Eds.), Catalysis by Transition Metal Sulphides, Ed. Technip, Paris, 2013, pp. 407-411 et références citées). Le coefficient de répartition macroscopique du platine, obtenu à partir de son profil déterminé par microsonde de Castaing, défini comme le rapport des concentrations platine au coeur de l'extrudé par rapport au bord de ce même extrudé, est compris entre 0,7 et 1,3, de préférence entre 0,8 et 1,2. La valeur de ce rapport, voisine de 1, témoigne de l'homogénéité de la répartition du platine dans le catalyseur.

La dispersion du(es) métal(ux) nobles du groupe VIII, déterminée par chimisorption, par exemple par titration H₂/O₂ ou par chimisorption du monoxyde de carbone, est comprise entre 10% et 100%, de préférence entre 20% et 100% et de manière encore plus préférée entre 30% et 100%.

Dans un mode de réalisation, la solution aqueuse de l'étape ii) ou une solution aqueuse différente de celle de l'étape ii) peut également comprendre les précurseurs des métaux des groupes IIIA, IVA et VIIB de la classification périodique des éléments et de préférence choisi parmi le gallium, l'indium, l'étain et le rhénium et de préférence choisi parmi l'indium, l'étain et le rhénium. Tous les précurseurs de tels métaux peuvent convenir.

Dans le cas où une solution différente de celle de l'étape ii) est utilisée, les dépôts des différents éléments sont réalisés de manière successive.

Selon une variante, lesdits précurseurs desdits métaux peuvent être imprégnés sur le support issu de l'étape i) séparément des précurseurs des métaux nobles du groupe VIII.

Lorsqu'au moins un métal des groupes IIIA, IVA et VIIB est ajouté séparément, il est préférable qu'il soit ajouté après le métal du groupe VIII. Dans ce cas, une deuxième étape optionnelle d'imprégnation d'au moins une solution aqueuse comprenant les précurseurs des métaux des groupes IIIA, IVA et VIIB peut avantageusement être mise en oeuvre après l'étape ii).

Le métal additionnel choisi parmi les métaux des groupes IIIA, IVA et VIIB peut être introduit par l'intermédiaire d'une solution aqueuse comprenant des composés choisis parmi les chlorures, les bromures et les nitrates des métaux des groupes IIIA, IVA et VIIB. Par exemple dans le cas de l'indium, on utilise avantageusement le nitrate ou le chlorure et dans le cas du rhénium, on utilise avantageusement l'acide perrhénique. Le métal additionnel choisi parmi les métaux des groupes IIIA, IVA et VIIB peut également être introduit par l'intermédiaire d'une solution comprenant au moins un composé organique choisi dans le groupe constitué par les complexes dudit métal, et de préférence les complexes polycétoniques du métal et les hydrocarbylmétaux choisis parmi les alkyles, les cycloalkyles, les aryles, les alkylaryles et les arylalkyles de métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel choisi parmi les métaux des groupes IIIA, IVA et VIIB est introduit avant le métal du groupe VIII, le composé du métal IIIA, IVA et/ou VIIB utilisé est généralement choisi dans le groupe constitué par l'halogénure, le nitrate, l'acétate, le tartrate, le carbonate et l'oxalate du métal. L'introduction est alors avantageusement effectuée en solution aqueuse comprenant lesdits composés. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.

De préférence, le ou les dépôts sont réalisés de manière à déposer sur ledit support une teneur en métaux des groupes IIIA, IVA et VIIB comprise entre 0,01 à 2%, et de préférence entre 0,05 à 1% poids par rapport à la masse totale dudit catalyseur.

Au moins une étape de séchage peut avantageusement être mise en oeuvre après la ou les étapes d'imprégnation, et de préférence après l'étape ii). Ladite étape de séchage est avantageusement effectuée par toute technique connue de l'homme du métier.

De préférence, le séchage est effectué sous flux d'air. Ledit séchage peut également être effectué sous flux de tout gaz oxydant, réducteur ou inerte. De préférence, le séchage est avantageusement effectué à une température comprise entre 50 et 180°C, de manière préférée entre 60 et 150°C et de manière très préférée entre 80 et 130°C.

Au moins une étape de calcination peut avantageusement être mise en oeuvre après la ou les étapes d'imprégnation, et de préférence après l'étape ii) et de manière préférée après au moins une étape de séchage.

Ladite étape de calcination est avantageusement réalisée en présence d'oxygène moléculaire, par exemple en effectuant un balayage d'air, à une température avantageusement supérieure à 200°C et inférieure ou égale à 1100°C. Ladite étape de calcination peut avantageusement être effectuée en lit traversé, en lit léché ou en atmosphère statique. Par exemple, le four utilisé peut être un four rotatif tournant ou être un four vertical à couches traversées radiales. De préférence, ladite étape de calcination est effectuée entre plus d'une heure à 200°C à moins d'une heure à 1100°C.

Dans le cas ou plusieurs étapes successives d'imprégnation sont réalisées, des étapes de séchage et/ou calcination intermédiaires et/ou une réduction peuvent avantageusement être mises en oeuvre entre les étapes d'imprégnations successives des différents métaux.

Avant son utilisation dans le procédé selon l'invention, le catalyseur obtenu à l'issue du procédé de préparation selon l'invention est de préférence soumis à une étape de réduction. Cette étape de réduction est avantageusement réalisée par un traitement sous hydrogène à une température comprise entre 150°C et 650°C et une pression totale comprise entre 0,1 et 25 MPa. Par exemple, une réduction consiste en un palier à 150°C de deux heures puis une montée en température jusqu'à 450°C à la vitesse de 1°C/min puis un palier de deux heures à 450°C; durant toute cette étape de réduction, le débit d'hydrogène est de 1000 normaux m³ d'hydrogène par tonne catalyseur et la pression totale maintenue constante à 0,2 MPa. Toute méthode de réduction *ex-situ* peut avantageusement être envisagée. Une réduction préalable du catalyseur final *ex situ,* sous courant d'hydrogène, peut être mise en oeuvre, par exemple à une température de 450°C à 600°C, pendant une durée de 0,5 à 4 heures.

Ledit catalyseur comprend également avantageusement du soufre. Dans le cas où le catalyseur de l'invention contient du soufre, celui-ci peut être introduit à n'importe quelle étape de la préparation du catalyseur: avant ou après étape de mise en forme, et/ou séchage et/ou calcination, avant et/ou après l'introduction du ou des métaux cités précédemment, ou encore par sulfuration *in situ* et ou *ex situ* avant la réaction catalytique. Dans le cas d'une sulfuration *in situ,* la réduction, si le catalyseur n'a pas été préalablement réduit, intervient avant la sulfuration. Dans le cas d'une sulfuration *ex situ,* on effectue également la réduction puis la sulfuration. La sulfuration s'effectue de préférence en présence d'hydrogène en utilisant tout agent sulfurant bien connu de l'homme de métier, tel que par exemple le sulfure de diméthyle ou le sulfure d'hydrogène.

Les catalyseurs selon l'invention se présentent sous différentes formes et dimensions. Ils sont utilisés en général sous la forme d'extrudés cylindriques et/ou polylobés tels que bilobés, trilobés, polylobés de forme droite et/ou torsadée, mais peuvent éventuellement être fabriqués et employés sous la forme de poudres concassées, de tablettes, d'anneaux, de billes et/ou de roues. De préférence, les catalyseurs mis en oeuvre dans le procédé selon l'invention ont la forme de sphères ou d'extrudés. Avantageusement le catalyseur se présente sous forme d'extrudés d'un diamètre compris entre 0,5 et 5 mm et plus particulièrement entre 0,7 et 2,5 mm. Les formes peuvent être cylindriques (qui peuvent être creuses ou non) et/ou cylindriques torsadés et/ou multilobées (2, 3, 4 ou 5 lobes par exemple) et/ou anneaux. La forme multilobée est avantageusement utilisée de manière préférée. Le dépôt du métal ne change pas la forme du support.

Le procédé de préparation selon l'invention permet donc l'obtention d'un catalyseur bifonctionnel comprenant une phase acide à base de zéolithe IZM-2 et une fonction hydrogénante à base de métaux nobles du groupe VIII permettant à la fois une localisation préférentielle de ladite fonction hydrogénante sur la surface et/ou dans la microporosité de la zéolithe IZM-2 et une répartition homogène de la fonction hydrogénante sur ledit catalyseur.

Un autre objet de l'invention concerne le catalyseur comprenant une fonction acide constituée par de la zéolithe IZM-2, une fonction hydrogénante comprenant au moins un métal noble du groupe VIII de la classification périodique choisi parmi le platine et le palladium et une matrice, obtenu par le procédé selon l'invention.

Conformément à l'invention, le métal du groupe VIII est localisé préférentiellement dans les cristaux et/ou à la surface des cristaux de la zéolithe IZM-2 et le métal du groupe VIII est réparti de manière homogène sur ledit catalyseur.

Ledit catalyseur préparé selon l'invention peut comprendre avantageusement au moins un métal additionnel choisi dans le groupe formé par les métaux des groupes IIIA, IVA et VIIB de la classification périodique des éléments et de préférence choisi parmi le gallium, l'indium, l'étain et le rhénium. Ledit métal additionnel est de préférence choisi parmi l'indium, l'étain et le rhénium.

Ledit catalyseur comprend également avantageusement du soufre.

Ledit catalyseur préparé selon l'invention comprend plus particulièrement, et de préférence est constitué de:
- de 1 à 50%, de préférence de 2 à 45% et de manière encore plus préférée de 3 à 40% poids de la zéolithe IZM-2 selon l'invention,
- de 0,01 à 4%, de préférence de 0,05 à 2% poids d'au moins un métal du groupe VIII de la classification périodique des éléments, de préférence le platine,
- éventuellement de 0,01 à 2%, de préférence de 0,05 à 1% poids d'au moins un métal additionnel choisi dans le groupe formé par les métaux des groupes IIIA, IVA et VIIB,
- éventuellement une teneur en soufre, de préférence telle que le rapport du nombre de moles de soufre sur le nombre de moles de(s) métal(ux) du groupe VIII soit compris entre 0,3 et 20,
- au moins une matrice, de préférence l'alumine, assurant le complément à 100% dans le catalyseur.

### Le procédé d'isomérisation

La présente invention a également pour objet un procédé d'isomérisation d'une charge parraffinique, ledit procédé comprenant la mise en contact de ladite charge paraffinique avec au moins ledit catalyseur selon l'invention présent dans un réacteur catalytique.

Conformément à l'invention, ladite charge paraffinique utilisée dans le procédé selon l'invention est produite à partir de ressources renouvelables.

Les paraffines de ladite charge paraffinique présentent un nombre d'atomes de carbone compris entre 9 et 25, de préférence compris entre 10 et 25 et de manière très préférée entre 10 et 22. La teneur en paraffines dans ladite charge mise en oeuvre dans le procédé selon l'invention est avantageusement supérieure à 90% poids, de préférence supérieure à 95% poids, de manière encore plus préférée supérieure à 98% poids. Au sein desdites parrafines, le pourcentage massique d'isoparaffines est inférieur à 15%, de manière préférée inférieur à 10% et de manière très préférée inférieur à 5%.

De préférence, ladite charge paraffinique est produite à partir de ressources renouvelables choisies parmi les huiles végétales, les huiles d'algues ou algales, les huiles de poissons et les graisses d'origine végétale ou animale, ou des mélanges de telles charges.

Lesdites huiles végétales peuvent avantageusement être brutes ou raffinées, totalement ou en partie, et issues des végétaux choisis parmi le colza, le tournesol, le soja, le palmier, l'olive, la noix de coco, le coprah, le ricin, le coton, les huiles d'arachides, de lin et de crambe et toutes les huiles issues par exemple du tournesol ou du colza par modification génétique ou hybridation, cette liste n'étant pas limitative. Lesdites graisses animales sont avantageusement choisies parmi le lard et les graisses composées de résidus de l'industrie alimentaire ou issus des industries de la restauration. Les huiles de fritures, les huiles animales variées comme les huiles de poisson, le suif, le saindoux peuvent également être utilisées.

Les ressources renouvelables à partir desquelles est produite la charge paraffinique utilisée dans le procédé selon l'invention contiennent essentiellement des structures chimiques de type triglycérides que l'homme du métier connait également sous l'appellation tri ester d'acides gras ainsi que des acides gras libres, dont les chaînes grasses contiennent un nombre d'atomes de carbone compris entre 9 et 25.

La structure et la longueur de chaîne hydrocarbonée de ces derniers est compatible avec les hydrocarbures présents dans le gazole et le kérosène, c'est à dire la coupe distillats moyens. Un tri ester d'acide gras est ainsi composé de trois chaînes d'acides gras. Ces chaînes d'acide gras sous forme de tri ester ou sous forme d'acide gras libres, possèdent un nombre d'insaturations par chaîne, également appelé nombre de doubles liaisons carbone-carbone par chaîne, généralement compris entre 0 et 3 mais qui peut être plus élevé notamment pour les huiles issues d'algues qui présentent généralement un nombre d'insaturations par chaînes de 5 à 6.

Les molécules présentes dans lesdites ressources renouvelables utilisées dans la présente invention présentent donc un nombre d'insaturations, exprimé par molécule de triglycéride, avantageusement compris entre 0 et 18. Dans ces charges, le taux d'insaturation, exprimé en nombre d'insaturations par chaîne grasse hydrocarbonée, est avantageusement compris entre 0 et 6.

Les ressources renouvelables comportent généralement également différentes impuretés et notamment des hétéroatomes tels que l'azote. Les teneurs en azote dans les huiles végétales sont généralement comprises entre 1 ppm et 100 ppm poids environ, selon leur nature. Elles peuvent atteindre jusqu'à 1% poids sur des charges particulières.

Ladite charge paraffinique utilisée dans le procédé selon l'invention est avantageusement produite à partir de ressources renouvelables selon des procédés connus de l'homme du métier. Une voie possible est la transformation catalytique desdites ressources renouvelables en effluent paraffinique désoxygéné en présence d'hydrogène et en particulier, l'hydrotraitement.

De préférence, ladite charge paraffinique est produite par hydrotraitement desdites ressources renouvelables. Ces procédés d'hydrotraitement de ressources renouvelables sont déjà bien connus et sont décrits dans de nombreux brevets. A titre d'exemple, ladite charge paraffinique utilisée dans le procédé selon l'invention peut avantageusement être produite, de préférence par hydrotraitement puis par séparation gaz/liquide, à partir desdites ressources renouvelables comme dans le brevet FR 2 910 483 ou dans le brevet FR 2 950 895.

Ladite charge paraffinique utilisée dans le procédé selon l'invention peut aussi être une charge paraffinique produite par un procédé mettant en jeu une étape de valorisation par la voie Fischer-Tropsch. Dans le procédé Fischer-Tropsch, le gaz de synthèse (CO+H₂) est transformé catalytiquement en produits oxygénés et en hydrocarbures essentiellement linéaires sous forme gazeuse, liquide ou solide. Lesdits produits obtenus constituent la charge du procédé selon l'invention. Le gaz de synthèse (CO+H₂) est avantageusement produit à partir de gaz naturel, de charbon, de biomasse, de toute source de composés hydrocarbonés ou d'un mélange de ces sources. Ainsi, les charges paraffiniques obtenues, selon un procédé de synthèse Fischer-Tropsch, à partir d'un gaz de synthèse (CO+H₂) produit à partir de ressources renouvelables, de gaz naturel ou de charbon peuvent être utilisées dans le procédé selon l'invention. De préférence, ladite charge paraffinique produite par synthèse Fischer-Tropsch et utilisée dans le procédé selon l'invention comprend majoritairement des n-paraffines. Ainsi, ladite charge comprend une teneur en n-paraffines supérieure à 60% poids par rapport à la masse totale de ladite charge. Ladite charge peut également comprendre une teneur en produits oxygénés de préférence inférieure à 10% poids, une teneur en insaturés, c'est-à-dire de préférence en produits oléfiniques, de préférence inférieure à 20% en poids et une teneur en iso-paraffines de préférence inférieure à 10% en poids par rapport à la masse totale de ladite charge.

De manière très préférée, ladite charge comprend une teneur en n-paraffines supérieure à 70% poids et de manière encore plus préférée supérieure à 80% poids par rapport à la masse totale de ladite charge. Les paraffines de ladite charge paraffinique présentent un nombre d'atomes de carbone compris entre 9 et 25, de préférence compris entre 10 et 25 et de manière très préférée entre 10 et 22.

De préférence, ladite charge paraffinique produite par synthèse Fischer-Tropsch est exempte d'impuretés hétéroatomiques telles que, par exemple, le soufre, l'azote ou des métaux.

Ledit procédé d'isomérisation est mis en oeuvre généralement selon les conditions opératoires suivantes :
- une température de 200°C à 500°C, de préférence de 210°C à 450°C et de manière encore plus préférée de 220°C à 430°C ;
- une pression partielle d'hydrogène de 0,3 à 5,5 MPa, de préférence de 0,4 et 4,8 MPa;
- une pression totale de 0,45 à 7 MPa, de préférence de 0,6 à 6 MPa ; et
- une vitesse spatiale d'alimentation, exprimée en kilogramme de charge introduite par kilogramme de catalyseur et par heure, de 0,25 à 30 h⁻¹, de préférence de 1 à 10 h⁻¹ et de manière encore préférée de 2 à 6 h⁻¹.

Les exemples suivants illustrent l'invention sans toutefois en limiter la portée.

### Exemples

### Exemple 1: synthèse de la zéolithe IZM-2

La zéolithe IZM-2 a été synthétisée conformément à l'enseignement du brevet FR 2 918 050 B. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante : 1 SiO₂; 0,0060 Al₂O₃; 0,1666 Na₂O; 0,1666 1,6bis(méthylpiperidinium)hexane; 33,3333 H₂O. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois avec une solution fraiche de nitrate d'ammonium, puis le solide est filtré, lavé à l'eau déionisée et séché en étuve une nuit à 100°C. Enfin, pour obtenir la zéolithe sous sa forme acide (protonée H⁺) on réalise une étape de calcination à 550°C durant dix heures (rampe de montée en température de 2°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. Des caractérisations au moyen des méthodes RMN de I'²⁷ Al, fluorescence X et ICP permettent d'accéder aux résultats suivants pour l'IZM-2 :
- pourcentage pondéral d'atomes d'aluminium hexacoordinés Al^{VI} : 5%,
- rapport du nombre de moles de silicium divisé par le nombre de moles d'aluminium de réseau, en mole/mole, Si/Al : 72,
- rapport du nombre de moles de sodium divisé par le nombre de moles d'aluminium de réseau, en mole/mole, Na/AI : 0,03.

### Exemple 2: préparation du support IZM-2/alumine.

Le support IZM-2/alumine est obtenu par malaxage et extrusion de la zéolithe IZM-2 préparée dans l'exemple 1 avec un gel d'alumine de type GA7001 fourni par la société AXENS. La pâte malaxée est extrudée au travers d'une filière trilobes de diamètre 1,8 mm. Après séchage en étuve une nuit à 110°C, les extrudés sont calcinés à 500°C durant deux heures (rampe de montée en température de 5°C/min) en lit traversé sous air sec (2 normaux litres par heure et par gramme de solide). La teneur pondérale de la zéolithe IZM-2 sur le support après calcination est de 25% poids.

### Exemple 3 (conforme à l'invention) : préparation du catalyseur d'isomérisation A.

Le catalyseur A est un catalyseur comprenant une zéolithe IZM-2, du platine, et une matrice alumine. Ce catalyseur est préparé par imprégnation en excès du support IZM-2/alumine décrit dans l'exemple 2 par une solution aqueuse contenant du chlorure de platine tétramine Pt(NH₃)₄Cl₂ et du nitrate d'ammonium NH₄NO₃. Le rapport molaire entre le nitrate d'ammonium et le platine est de 120. La concentration en chlorure de platine tétramine dans la solution est de 1,54 10⁻³ mol/l et celle en nitrate d'ammonium est de 1,84 10⁻¹ mol/l.

On utilise 20 grammes de support dont on remplit le volume poreux par de l'eau distillée et on laisse le solide à maturer durant une heure à température ambiante. Le solide est ensuite immergé dans 160 ml de la solution d'imprégnation précédemment décrite dans un erlenmeyer puis l'ensemble est mis sur agitation sur une table d'agitation (100 tours/min) à température ambiante durant 24 heures. La solution d'imprégnation est ensuite soutirée et le solide est rincé avec 320 ml d'eau distillée. Le solide est ensuite mis à sécher en étuve ventilée durant la nuit à 110°C et on effectue finalement une étape de calcination sous débit d'air sec (2 normaux litres par heure et par gramme de solide) dans un four tubulaire dans les conditions suivantes :
- montée de la température à l'ambiante à 500°C à 5°C/min ;
- palier de deux heures à 500°C ;
- descente à l'ambiante.

La teneur en Pt mesurée par FX sur le catalyseur calciné est de 0,19% en poids, sa dispersion mesurée par titrage H₂/O₂ est de 81%, son coefficient de répartition mesuré par microsonde de Castaing de 0,98. Des analyses locales sur le catalyseur examiné en microscopie électronique à transmission sont effectuées sur différentes zones de la matrice alumine et sur divers agglomérats de cristallites d'IZM-2.

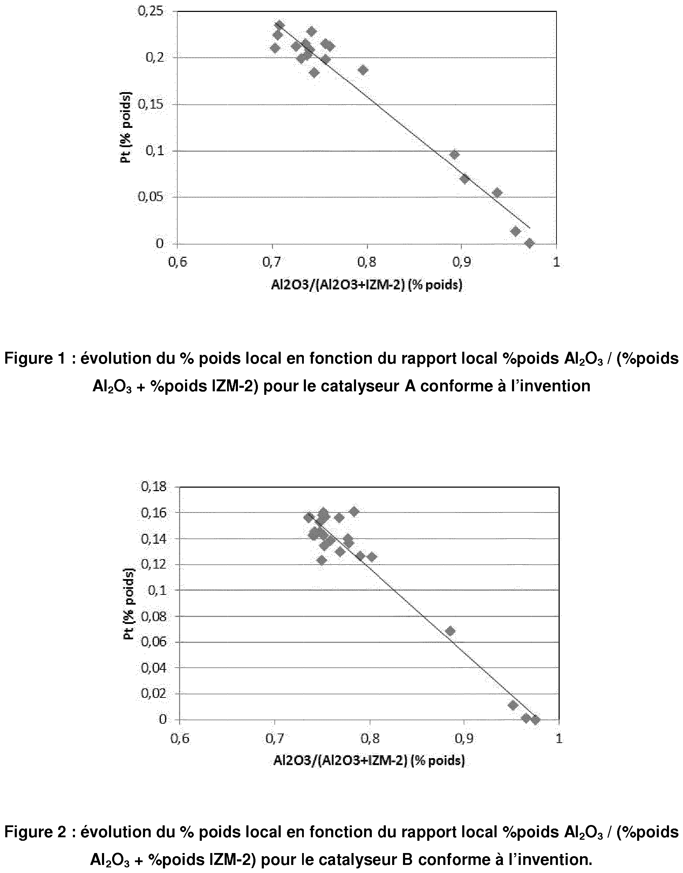

La figure 1 représente l'évolution du pourcentage poids local en platine en fonction du rapport %poids Al₂O₃/(%poids Al₂O₃+%poids IZM-2) local obtenue par microsonde de Castaing. Localement le pourcentage poids en Pt diminue avec l'augmentation de la quantité d'alumine par rapport à la quantité d'IZM-2 ce qui traduit un dépôt préférentiel du platine sur la zéolithe IZM-2. On observe ainsi que pour des rapports %poids Al₂O₃/(%poids Al₂O₃+%poids IZM-2) qui tendent vers 1, c'est-à-dire pour des zones analysées ne contenant pas de zéolithe IZM-2, le % poids en platine tend vers une valeur nulle.

### Exemple 4 (conforme à l'invention) : préparation du catalyseur d'isomérisation B.

Le catalyseur B est un catalyseur comprenant une zéolithe IZM-2, du platine, et une matrice alumine. Ce catalyseur est préparé par imprégnation en excès du support IZM-2/alumine préparé dans l'exemple 2 par une solution aqueuse contenant du chlorure de platine tétramine Pt(NH₃)₄Cl₂ et du nitrate d'ammonium NH₄NO₃. Le rapport molaire entre le nitrate d'ammonium et le platine est de 120. La concentration en chlorure de platine tétramine dans la solution est de 9,62 10⁻⁴ mol/l et celle en nitrate d'ammonium est de 1,15 10⁻¹ mol/l.

On utilise 20 grammes de support dont on remplit le volume poreux par de l'eau distillée et on laisse le solide à maturer durant une heure à température ambiante. Le solide est ensuite immergé dans 160 ml de la solution d'imprégnation précédemment décrite dans un erlenmeyer puis l'ensemble est mis sur agitation sur une table d'agitation (100 tours/min) à température ambiante durant 24 heures. La solution d'imprégnation est ensuite soutirée et le solide est rincé avec 320 ml d'eau distillée. Le solide est ensuite mis à sécher en étuve ventilée durant la nuit à 110°C et on effectue finalement une étape de calcination sous débit d'air sec (2 normaux litres par heure et par gramme de solide) dans un four tubulaire dans les conditions suivantes :
- montée de la température à l'ambiante à 500°C à 5°C/min ;
- palier de deux heures à 500°C ;
- descente à l'ambiante.

La teneur en Pt mesurée par FX sur le catalyseur calciné est de 0,14% en poids, sa dispersion mesurée par titrage H₂/O₂ est de 76%, son coefficient de répartition mesuré par microsonde de Castaing de 0,98.

La figure 2 représente l'évolution du pourcentage poids local en platine en fonction du rapport %poids Al₂O₃/(%poids Al₂O₃+%poids IZM-2) local obtenue par microsonde de Castaing. Tout comme pour le catalyseur A on note que localement le pourcentage poids en Pt diminue avec l'augmentation de la quantité d'alumine par rapport à la quantité d'IZM-2 ce qui traduit un dépôt préférentiel du platine sur la zéolithe IZM-2.

### Exemple 5 (non conforme à l'invention) : préparation du catalyseur d'isomérisation C.

Le catalyseur C est un catalyseur comprenant une zéolithe IZM-2, du platine, et une matrice alumine. Ce catalyseur est préparé par imprégnation à sec du support IZM-2/alumine préparé dans l'exemple 2 par une solution aqueuse contenant du chlorure de platine tétramine Pt(NH₃)₄Cl₂. Il n'y a pas de sel d'ammonium dans la solution d'imprégnation. On utilise typiquement 20 grammes de support que l'on imprègne à sec en drageoir. Après imprégnation le solide est laissé à maturer durant au moins cinq heures en air laboratoire puis mis à sécher une nuit en étuve à 110°C et on effectue finalement une étape de calcination sous débit d'air sec (2 normaux litres par heure et par gramme de solide) dans un four tubulaire dans les conditions suivantes :
- montée de la température à l'ambiante à 500°C à 5°C/min ;
- palier de deux heures à 500°C ;
- descente à l'ambiante.

La teneur en Pt mesurée par FX sur le catalyseur calciné est de 0,2% en poids, sa dispersion mesurée par titrage H₂/O₂ est de 38%, son coefficient de répartition mesuré par microsonde de Castaing de 0,62. Le Pt n'est donc pas réparti de manière homogène dans le support. La non homogénéité de la répartition du Pt sur le support ne permet pas l'utilisation de la microsonde de Castaing pour déterminer la localisation du Pt sur le support. Cependant, les analyses du catalyseur par microscopie électronique en transmission à balayage sur appareil JEOL JEM2100F associé à un détecteur annulaire à grand angle (STEM-HAADF) mettent en évidence la localisation des particules de platine sur les cristallites de zéolithe IZM-2.

### Exemple 6 (non conforme à l'invention) : préparation du catalyseur d'isomérisation D.

Le catalyseur D est un catalyseur comprenant une zéolithe IZM-2, du platine, et une matrice alumine. Ce catalyseur est préparé par imprégnation en excès du support IZM-2/alumine préparé dans l'exemple 2 par une solution aqueuse contenant de l'acide hexachloroplatinique. La concentration en acide hexachloroplatinique dans la solution est de 2,55 10⁻³ mol/l.

On utilise 20 grammes de support dont on remplit le volume poreux par de l'eau distillée et on laisse le solide à maturer durant une heure à température ambiante. Le solide est ensuite immergé dans 80 ml d'une solution d'acide chlorhydrique HCl de concentration 3,52 10⁻¹ mol/l dans un erlenmeyer puis l'ensemble est mis sur agitation sur une table d'agitation (100 tours/min) à température ambiante durant une heure. La solution d'acide chlorhydrique est ensuite soutirée puis le solide est immergé dans 80 ml de la solution d'acide hexachloroplatinique précédemment décrite puis l'ensemble est mis sur agitation sur une table d'agitation (100 tours/min) à température ambiante durant 24 heures. La solution d'imprégnation est ensuite soutirée et le solide est rincé avec 160 ml d'eau distillée. Le solide est ensuite mis à sécher en étuve ventilée durant la nuit à 110°C et on effectue finalement une étape de calcination sous débit d'air sec (2 normaux litres par heure et par gramme de solide) dans un four tubulaire dans les conditions suivantes :
- montée de la température à l'ambiante à 500°C à 5°C/min ;
- palier de deux heures à 500°C ;
- descente à l'ambiante.

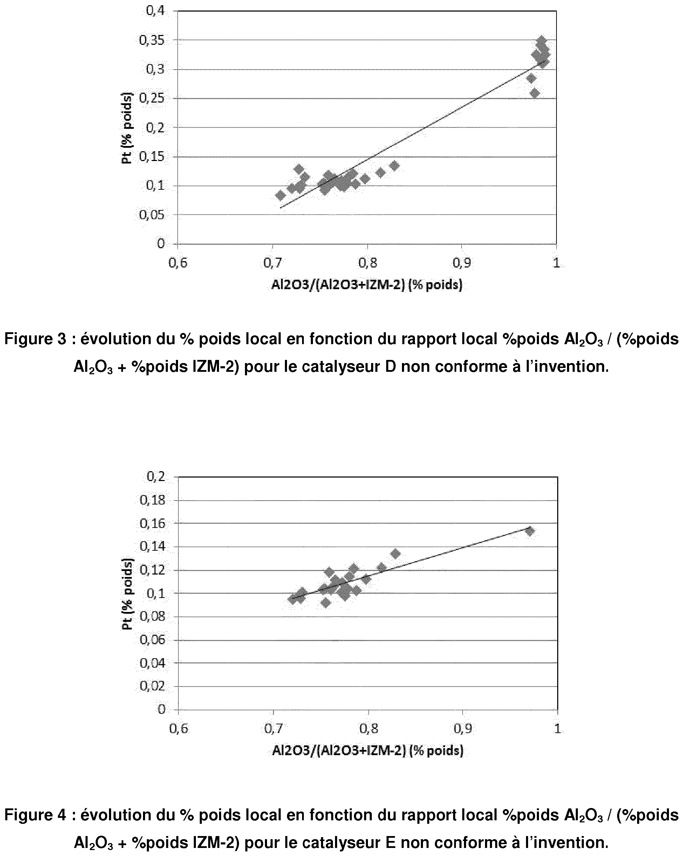

La teneur en Pt mesurée par FX sur le catalyseur calciné est de 0,20% en poids, sa dispersion mesurée par titrage H₂/O₂ est de 83%, son coefficient de répartition mesuré par microsonde de Castaing de 0,95. La figure 3 représente l'évolution du pourcentage poids local en platine en fonction du rapport %poids Al₂O₃/(%poids Al₂O₃+%poids IZM-2) local obtenue par microsonde de Castaing. Contrairement aux catalyseurs A et B, on note que localement le pourcentage poids en Pt augmente avec l'augmentation de la quantité d'alumine par rapport à la quantité d'IZM-2 ce qui traduit un dépôt préférentiel du platine sur l'alumine.

### Exemple 7 (non conforme à l'invention) : préparation du catalyseur d'isomérisation E.

Le catalyseur E est un catalyseur comprenant une zéolithe IZM-2, du platine, et une matrice alumine. Ce catalyseur est préparé par imprégnation en excès du support IZM-2/alumine préparé dans l'exemple 2 par une solution aqueuse contenant de l'acide hexachloroplatinique. La concentration en acide hexachloroplatinique dans la solution est de 1,28 10⁻³ mol/l.

On utilise 20 grammes de support dont on remplit le volume poreux par de l'eau distillée et on laisse le solide à maturer durant une heure à température ambiante. Le solide est ensuite immergé dans 80 ml d'une solution d'acide chlorhydrique HCl de concentration 3,52 10⁻¹ mol/l dans un erlenmeyer puis l'ensemble est mis sur agitation sur une table d'agitation (100 tours/min) à température ambiante durant une heure. La solution d'acide chlorhydrique est ensuite soutirée puis le solide est immergé dans 80 ml de la solution d'acide hexachloroplatinique précédemment décrite puis l'ensemble est mis sur agitation sur une table d'agitation (100 tours/min) à température ambiante durant 24 heures. La solution d'imprégnation est ensuite soutirée et le solide est rincé avec 160 ml d'eau distillée. Le solide est ensuite mis à sécher en étuve ventilée durant la nuit à 110°C et on effectue finalement une étape de calcination sous débit d'air sec (2 normaux litres par heure et par gramme de solide) dans un four tubulaire dans les conditions suivantes :
- montée de la température à l'ambiante à 500°C à 5°C/min ;
- palier de deux heures à 500°C ;
- descente à l'ambiante.

La teneur en Pt mesurée par FX sur le catalyseur calciné est de 0,10% en poids, sa dispersion mesurée par titrage H₂/O₂ est de 80%, son coefficient de répartition mesuré par microsonde de Castaing de 1,02.

La figure 4 représente l'évolution du pourcentage poids local en platine en fonction du rapport %poids Al₂O₃/(%poids Al₂O₃+%poids IZM-2) local obtenue par microsonde de Castaing. Tout comme pour le catalyseur D, on note que localement le pourcentage poids en Pt augmente avec l'augmentation de la quantité d'alumine par rapport à la quantité d'IZM-2 ce qui traduit un dépôt préférentiel du platine sur l'alumine.

### Exemple 8 : évaluation des propriétés catalytiques des catalyseurs A, et B conformes à l'invention et C, D et E non conformes à l'invention, en isomérisation d'une charge paraffinique.

Les catalyseurs ont été testés en isomérisation d'une charge paraffinique composée par du n-hexadécane. Les tests ont été effectués dans une micro-unité mettant en oeuvre un réacteur lit fixe et travaillant en courant descendant sans recyclage. L'analyse des effluents hydrocarbonés est effectuée en ligne par chromatographie en phase gazeuse. Une fois chargé dans l'unité, le catalyseur subit une première étape de séchage sous azote dans les conditions suivantes :
- débit d'azote: 2 normaux litres par heure et par gramme de catalyseur,
- pression totale: 0,1 MPa,
- rampe de montée en température de l'ambiante à 150°C: 5°C/min,
- palier à 150°C de 30 minutes.

Après séchage l'azote est remplacé par l'hydrogène et une étape de réduction sous débit d'hydrogène pur est effectuée ensuite dans les conditions suivantes:
- débit d'hydrogène: 5 normaux litres par heure et par gramme de catalyseur,
- pression totale: 1,1 MPa,
- rampe de montée en température de 150 à 450°C: 5°C/min,
- palier à 450°C de 1 heure.

Après étape de réduction, la température est descendue à 230°C, et le catalyseur est mis en contact du n-hexadécane dans les conditions suivantes :
- vitesse spatiale d'alimentation de 2 grammes de n-hexadécane par heure et par gramme de catalyseur,
- rapport molaire hydrogène sur n-hexadécane de 10,
- pression totale de 1,1 MPa.

La conversion est modifiée en faisant varier la température; et à chaque palier de température deux analyses de l'effluent sont effectuées, ce qui permet de calculer les performances catalytiques et de vérifier la stabilité des performances catalytiques pour ledit palier de température. Typiquement on fait varier la température entre 230 et 350°C par palier de température de 5°C. L'analyse des effluents s'effectue intégralement par le biais d'un système GC en ligne. La température nécessaire pour atteindre 50% de conversion fait office de descripteur de l'activité du catalyseur alors que le rendement maximal obtenu en isomères de l'hexadécane fait office de descripteur des propriétés isomérisantes du catalyseur.

Le tableau 1 reporte ainsi les performances catalytiques des catalyseurs en hydroconversion du n-hexadécane. On observe que les catalyseurs A et B conformes préparés selon le procédé de l'invention présentent une activité nettement plus importante que les catalyseurs non conformes à l'invention : la conversion de 50% du n-hexadécane est obtenue pour des températures plus basses de typiquement 13 à 15°C par rapport aux catalyseurs non conformes D et E, et typiquement 21 à 22°C par rapport au catalyseur C. De plus les catalyseurs conformes à l'invention A et B permettent d'atteindre les rendements maximaux en isomères du n-hexadécane les plus élevés. Les catalyseurs A et B préparés selon le procédé de l'invention présentent donc à la fois les meilleures activités et les meilleures propriétés isomérisantes.

**Tableau 1 : performances catalytiques des catalyseurs A, B, C, D, E en hydroconversion du n-hexadécane.**

| Catalyseur | A (conforme) | B (conforme) | C (non conforme) | D (non conforme) | E (non conforme) |
|---|---|---|---|---|---|
| Température à 50% conversion (°C) | 254 | 255 | 276 | 269 | 268 |
| Rendement max en isomères (%poids) | 87 | 87 | 86 | 84 | 85 |

## Revendications

1. Procédé de préparation d'un catalyseur bifonctionnel comprenant une fonction acide constituée par de la zéolithe IZM-2, une fonction hydrogénante comprenant au moins un métal noble du groupe VIII de la classification périodique, choisi parmi le platine et le palladium et une matrice, ledit procédé comprenant au moins les étapes suivantes :
i) une étape de préparation du support du catalyseur par mise en forme de la zéolithe IZM-2 avec une matrice,
ii) une étape de dépôt d'au moins un métal noble du groupe VIII de la classification périodique par imprégnation du support préparé à l'étape i) par une solution aqueuse comprenant
• au moins un composé ammoniaqué choisi parmi les sels de platine (II) tétramines de formule Pt(NH₃)₄(OH)₂, Pt(NH₃)₄(NO₃)₂ ou Pt(NH₃)₄X₂, les sels de platine (IV) hexamines de formule Pt(NH₃)₆X₄; les sels de platine (IV) halogénopentamines de formule (PtX(NH₃)₅)X₃; les sels de platine N-tétrahalogénodiamines de formule PtX₄(NH₃)₂; les composés halogénés de formule H(Pt(acac)₂X); les sels de palladium (II) de formule Pd(NH₃)₄SO₄ ou Pd(NH₃)₄X₂, X étant un halogène choisi parmi le chlore, le fluor, le brome et l'iode, et "acac" représentant le groupe acétylacétonate,
• et au moins un sel d'ammonium choisi parmi le nitrate d'ammonium NH₄NO₃, le chlorure d'ammonium NH₄Cl, l'hydroxyde d'ammonium NH₄OH, le bicarbonate d'ammonium NH₄HCO₃, l'acétate d'ammonium NH₄H₃C₂O₂ seul ou en mélange,
• le rapport molaire entre le sel d'ammonium et le métal noble étant compris entre 0,1 et 400.

2. Procédé selon la revendication 1 dans lequel l'étape i) est mise en oeuvre par malaxage - extrusion.

3. Procédé selon l'une des revendications 1 ou 2 dans lequel ladite matrice mise en oeuvre dans l'étape i) est l'alumine.

4. Procédé selon l'une des revendications 1 à 3 dans lequel le support obtenu à l'issu de l'étape i) est soumis à une étape de séchage effectuée à une température comprise entre 50 et 180°C.

5. Procédé selon l'une des revendications 1 à 4 dans lequel X est le chlore.

6. Procédé selon l'une des revendications 1 à 5 dans lequel l'imprégnation du support à l'étape ii) est effectuée par la méthode d'imprégnation dite "à sec" ou en excès de ladite solution.

7. Procédé selon l'une des revendications 1 à 6 dans lequel la solution aqueuse de l'étape ii) comprend des composés ammoniaqués choisis parmi les sels de platine (II) tétramines de formule Pt(NH₃)₄(OH)₂, Pt(NH₃)₄(NO₃), ou Pt(NH₃)₄X₂, les sels de platine (IV) hexamines de formule Pt(NH₃)₆X₄; les sels de platine (IV) halogénopentamines de formule (PtX(NH₃)₅)X₃; les sels de platine N-tétrahalogénodiamines de formule PtX₄(NH₃)₂; et les composés halogénés de formule H(Pt(acac)₂X); X et « acac » ayant la signification précitée.

8. Procédé selon la revendication 7 dans lequel ladite solution comprend des composés ammoniaqués choisis parmi les sels de platine (II) tétramines de formule Pt(NH₃)₄(OH)₂, Pt(NH₃)₄(NO₃), ou Pt(NH₃)₄X₂.

9. procédé selon l'une des revendications 1 à 8 dans lequel la solution aqueuse de l'étape ii) ou une solution aqueuse d'imprégnation différente de celle de l'étape ii) comprend les précurseurs des métaux des groupes IIIA, IVA et VIIB de la classification périodique des éléments choisi parmi le gallium, l'indium, l'étain et le rhénium.

10. Procédé selon les revendications 1 à 9 dans lequel le rapport molaire entre le sel d'ammonium et le métal noble est compris entre 0,3 et 150 dans la solution de l'étape ii).

11. Catalyseur comprenant une fonction acide constituée par de la zéolithe IZM-2, une fonction hydrogénante comprenant au moins un métal noble du groupe VIII de la classification périodique choisi parmi le platine et le palladium et une matrice, obtenu par le procédé selon l'une des revendications 1 à 10.

12. Procédé d'isomérisation d'une charge parraffinique présentant un nombre d'atomes de carbone compris entre 9 et 25, ledit procédé comprenant la mise en contact de ladite charge paraffinique avec au moins ledit catalyseur selon la revendication 11, ledit procédé étant mis en oeuvre à une température de 200°C à 500°C, une pression partielle d'hydrogène de 0,3 à 5,5 MPa, une pression totale de 0,45 à 7 MPa, et une vitesse spatiale d'alimentation, exprimée en kilogramme de charge introduite par kilogramme de catalyseur et par heure, de 0,25 à 30 h⁻¹.

13. Procédé selon la revendication 12 dans lequel ladite charge paraffinique est produite à partir de ressources renouvelables choisies parmi les huiles végétales, les huiles d'algues ou algales, les huiles de poissons et les graisses d'origine végétale ou animale, ou des mélanges de telles charges.

14. Procédé selon l'une des revendications 12 ou 13 dans lequel ladite charge paraffinique est produite par hydrotraitement desdites ressources renouvelables.

15. Procédé selon l'une des revendications 12 ou 13 dans lequel ladite charge paraffinique est une charge paraffinique produite par un procédé mettant en jeu une étape de valorisation par la voie Fischer-Tropsch.
